# EUROPEAN PATENT APPLICATION

(11) **EP 1 095 661 A1**
(43) Date of publication of application: **02.05.2001**
(21) Application number: 99203593.1
(22) Date of filing: 01.11.1999
(51) Int. Cl.: A61K 38/20, A61K 38/21, A61K 31/7088, A61L 27/00, A61F 2/00, A61P 31/04

(54) **Prevention and treatment of biomaterial-associated infections**

(71) Applicant: Academisch Ziekenhuis bij de Universiteit van Amsterdam, 1105 AZ Amsterdam (NL)
(72) Inventor: Boelens, Jaap Jan, 3512 KA Utrecht (NL); Dankert, Jaap, 1396 KG Baambrugge (NL); Van der Poll, Tom, 2341 KN Oegstgeest (NL); Zaat, Sebastianus Antonius Johannes, 1181 NG Amstelveen (NL)
(74) Representative: Van Someren, Petronella F. H. M.

(57) **Abstract**

The present invention relates to the use of one or more immunomodulators for the preparation of a medicament for the prevention and/or treatment of biomaterial-associated infections in humans or animals. The invention further relates to a biomaterial provided with one or more immunomodulators for the prevention and/or treatment of infections in humans or animals associated with the biomaterial, as well as to a biomedical device made of said biomaterial.

## Description

The present invention relates to the prevention and/or treatment of biomaterial-associated infections. In addition, the invention relates to biomaterials and biomedical devices that can be used to prevent or treat biomaterial-associated infections.

The implantation or insertion of biomedical devices, such as artificial heart valves, hips and knees, vascular prostheses and catheters, is virtually indispensable in modern medicine. Devices may be utilized briefly or intermittently (e.g. intravenous catheters) for months to years (e.g. intra-uterine devices), or permanently (e.g. artificial heart valves and hips).

A serious problem, however, associated with the use of the so-called biomaterials of which these devices are made, is the occurrence of bacterial infections. Frequencies of infection vary from 0.1-1% for intraocular lenses to more than 20% for catheters used in chronic ambulant peritoneal dialysis (CAPD). These infections cause considerable morbidity and mortality because biomaterial-associated infections (BAI) are rather resistant to the host defense and to antibiotic therapy. Therefore removal of an infected biomaterial is often required.

BAI is caused predominantly by coagulase-negative staphylococci, especially Staphylococcus epidermidis (40-75% of the infections). However, also Staphylococcus aureus (10 to 20%), and less frequently yeasts, such as Candida spp. and Malassezia spp., other yeasts or fungi spp., coryneforms, other gram-positive bacteria and various gram-negative bacteria may be the causative organisms of BAI. Gram-negative bacteria, such as Enterobacteriaceae and Pseudomonas are only a predominant cause of infection associated with urinary catheters or genitourinary implants, due to the location of the implanted biomaterial.

Until now, the pathogenesis of biomaterial-associated infections is poorly understood. A predominant role is attributed to the initial bacterial adherence as the first step in BAI, although alterations in host defense mechanisms in the vicinity of an implanted biomaterial have been frequently suggested.

During the last decades various strategies have been employed in the prevention of BAI, such as the use of strict protocols for preoperative skin preparation and postoperative wound care to reduce the risk for contamination of the wound and biomaterial and the use of systemic antibiotics for perioperative prophylaxis.

Additionally, regarding bacterial adherence as an essential step in the pathogenesis of BAI, the use of biomaterials with the ability to reduce bacterial adherence appeared to be a very attractive approach to prevent BAI. Therefore, surface-modified biomaterials and biomaterials impregnated/coated with antiseptic agents or antimicrobial(s) have been developed. Alternatively, catheters may be soaked in antibiotic solutions prior to their insertion. Although promising results regarding inhibition of bacterial adherence and duration of antibacterial activity of such novel biomaterials are often found in vitro, the use of such biomaterials did not always result in a reduced infection rate in vivo.

It is therefore the object of the present invention to provide a new means for preventing or treating biomaterial-associated infections.

In the research that led to the present invention it was surprisingly found that the infecting micro-organism is present within macrophages surrounding the biomaterial. Although these micro-organisms are phagocytosed by the cell, they are not killed inside that cell. This phenomenon was demonstrated with respect to S. epidermidis, which was able to persist in pericatheter macrophages. This persistence of S. epidermidis in pericatheter macrophages was found around 4 out of 4 commonly used biomaterials. Apparently, the local host defense is compromised due to the presence of the implanted biomaterials, resulting in macrophage deactivation and subsequent deficient intracellular killing. Sham operations, defined as similar surgical procedures and treatments, without the introduction of a biomaterial implant did not result in intracellular survival of S. epidermidis. Even when surgical wounds were deliberately infected with S. epidermidis, in sham operation experiments no intracellular survival in macrophages was observed, indicating that the presence of the biomaterial implant is a prerequisite for the observed phenomenon.

The present invention thus provides a solution to the heretofore unknown problem of intracellular survival of micro-organisms, in particular coagulase-negative staphylococci (such as Staphylococcus epidermidis) and S.aureus, in tissue in the vicinity of an implanted biomaterial.

The object of the present invention to provide a means for preventing or treating biomaterial-associated infection is therefore based on reversal of macrophage deactivation in the vicinity of an implanted biomaterial by means of the administration of one or more immunomodulators. The invention thus relates to the use of one or more immunomodulators for the preparation of a medicament for the prevention and/or treatment of biomaterial-associated infections in humans or animals.

The immunomodulator for use according to the invention is a compound that stimulates the host defense of the human or animal. In particular, the immunomodulator is a compound that stimulates intracellular killing of the infecting agent. Various known compounds are suited for this, in particular cytokines, their receptor antagonists, cytokine derivatives, or cytokine analogues. Cytokine derivatives are meant to encompass molecules having a structural similarity with existing cytokines, whereas analogues are understood to mean molecules having a similar function as existing cytokines. For the present invention it is preferred that the derivatives also have a similar function.

Immunomodulators suitable for use according to the present invention are interferon-γ (IFN-γ), interleukin-12 (IL-12), interleukin-15 (IL-15), interleukin-18 (IL-18), interleukin-1 receptor antagonist (IL-1 RA), etc. It was found that interferon-γ is particularly useful for inducing intracellular killing of S. epidermidis in macrophages. In addition, it was demonstrated according to this invention that interleukin-1 receptor type 1 gene-deficient mice are less susceptible to Staphylococcus epidermidis biomaterial-associated infection. Antagonists of this receptor have a similar activity.

Another class of immunomodulators are the CpG oligonucleotides. These oligonucleotides of about 8-20 nucleotides contain the unmethylated CpG motif, which was found to be present in bacterial DNA and to cause B cell proliferation, immunoglobulin secretion and monocyte cytokine secretion. These CpG oligonucleotides do also enhance the host defense against the biomaterial-associated infecting intracellular micro-organisms, like S. epidermidis.

The most commonly found biomaterial-associated infections are bacterial infections. The present invention is in particular directed to preventing and/or treating these infections, caused for example by bacteria like coagulase-negative staphylococci (such as Staphylococcus epidermidis), Staphylococcus aureus, Entercocci, Corynebacterium spp., Serratia spp., Klebsiella spp., Bacillus spp., Proteus spp., Enterobacter spp., Enterobacteriaceae spp., Pseudomonas spp., Acinetobacter spp., Clostridium spp. and Diftroide spp..

Although less frequently, biomaterial-associated infections may also be caused by yeasts. The present invention is therefore also directed to preventing and/or treating these yeast infections, caused for example by yeasts like Candida spp. or Malassezia spp, etc.

In a preferred embodiment thereof, the present invention relates to the use of interferon-γ for the preparation of a medicament for the prevention and/or treatment of biomaterial-associated infections in humans or animals. Interferon-γ is in particular suitable for treating and preventing infections that are caused by Staphylococcus epidermidis.

There are various manners for administration of the immunomodulators. For internal implants that are not accessible from outside the human or animal body, systemic ways of administering the immunomodulators are preferred, for example orally or through intravenous, subcutaneous or intramuscular injection.

For a topical way of administration, the biomaterial of which the biomedical device is made can be coated or impregnated with the immunomodulator(s). As an alternative, the biomaterial can be provided with a matrix or hydrogel that is soaked in a solution of the immunomodulator prior to implantation. This soaking leads to swelling of the matrix or hydrogel and (slow) release of the immunomodulator(s) inside the body.

In addition, the immunomodulators can be injected into the tissue surrounding the implant in cases where the implant is sufficiently close to the body surface.

When the immunomodulator is not part of the implant or administered systemically, it can be incorporated in dosage forms that lead to uptake of the immunomodulator through the skin or mucous membranes or through a wound or opening in the skin that forms the contact of the implant with the outside world. Such dosages forms can be plasters containing the immunomodulator, ointments, gels, creams, etc. Implants that can be accessed from outside the body are for example intravascular catheters, thorax drains, other drains or sutures.

The invention further relates to biomaterial provided with one or more immunomodulators for the prevention and/or treatment of infections in humans or animals associated with the biomaterial. Such biomaterial is for example coated with the immunomodulator, or the immunomodulator is comprised in a hydrogel coating of the biomaterial. Furthermore, the biomaterial itself may be impregnated with the immunomodulator, or the immunomodulator may be bound to a carrier applied on or in the biomaterial.

Implants or other biomedical or non-medical devices made of the thus coated or impregnated biomaterial are also part of the present invention. Such implants are for example artificial joints (hips, knees), heart valves, intubation tubes, pacemakers, left ventricular assistant devices, or artificial hearts. Other biomedical devices comprise vascular prostheses and catheters, intra-uterine devices, drains, intraocular lenses, artificial ears, skin, nose, or eyes, silicon implants in plastic surgery, penile and dental prostheses and piercings. Furthermore, biomaterials can be used as matrixes in tissue engineering or as implanted skin-extruders.

The term "biomaterial" as used herein is intended to mean any material, natural or man-made, that is introduced on or in the human or animal body, or comprises whole or part of a living structure or biomedical device which performs, augments, or replaces a natural function. Biomaterials can be subdivided into various groups, such as plastics (polymers), metals, various forms of carbon, cements and organic materials. Examples of polymers comprise but are not limited to polyamide, silicone elastomers, polycarbonate, polyurethane, nylon. Optionally such polymers may be modified, for example with polyvinylpyrrolidone (PVP). Examples of metals comprise but are not limited to aluminum, stainless steels, titanium. Cements are for example used in orthopedic surgery and by dentists or dental surgeons. Examples of cement materials comprise but are not limited to polymethylmethacrylate. Organic materials, such as catgut, are for example used for sutures.

The present invention is further illustrated by the following examples and figures, which are used to clarify the invention and are not intended to limit the scope thereof.

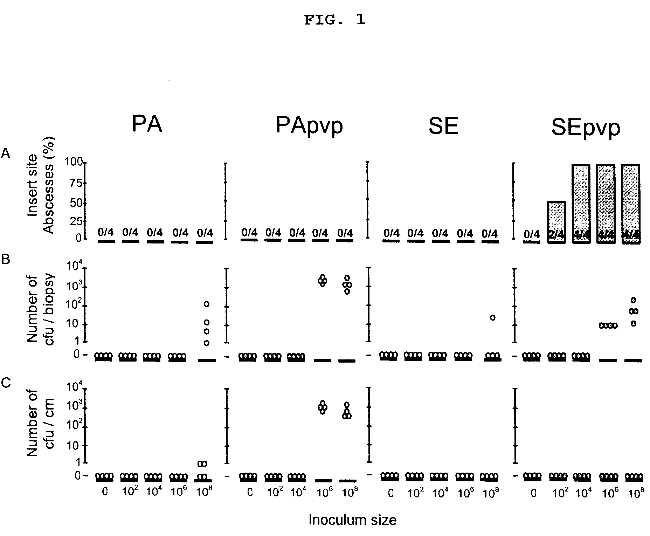

**Figure 1** demonstrates the frequency of subcutaneous abscesses along the inserted catheter segments (A), the number of cfu of S. epidermidis RP62a cultured from insertion site homogenates (B) and the number of adherent cfu of S. epidermidis RP62a cultured from subcutaneous implantation of PA, PApvp, SE and SEpvp (C) at 14 days (d) after challenge with various inocula of S. epidermidis RP62a. Four mice per group were tested.

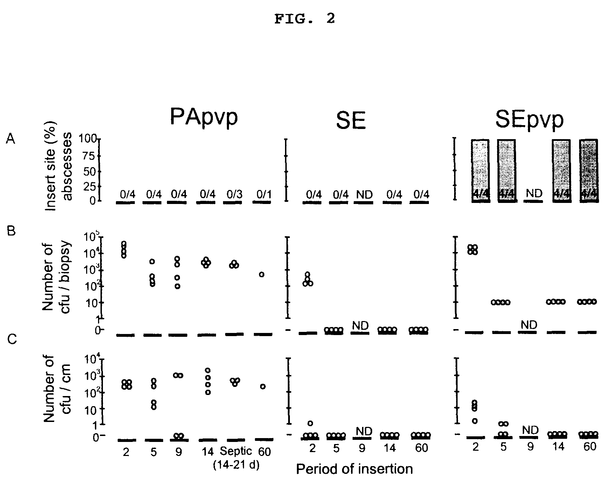

**Figure 2** shows the frequency of subcutaneous abscesses along the inserted catheter segments (A), the number of cfu of S. epidermidis RP62a cultured from insertion site homogenates (B) and the number of adherent S. epidermidis RP62a cultured from subcutaneous implantation of PApvp, SE and SEpvp (C) at various time points after challenge with 10⁶ cfu S. epidermidis RP62a. Four mice per group were tested at each time point. Three of 4 mice with PApvp to be terminated at 60 days became terminally ill between 14 and 21 days due to sepsis. They are represented as "septic mice" (ND= not determined).

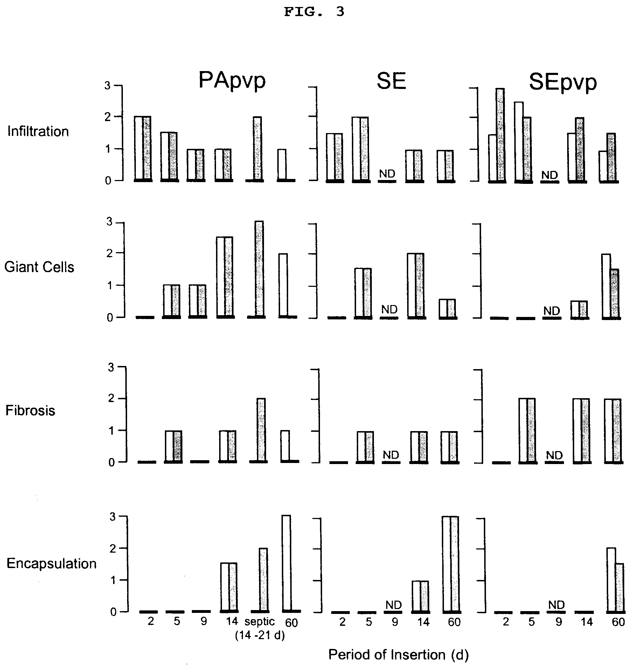

**Figure 3** demonstrates the mean scores of infiltration, foreign body giant cells, fibrosis and encapsulation (features characteristic for the implantation of a foreign body) at various time points after subcutaneous implantation of PApvp, SE or SEpvp, after challenge with saline or 10⁶ cfu S. epidermidis RP62a. Four mice per group were tested at each time point (empty bars: saline; filled bars: S. epidermidis; ND=not determined).

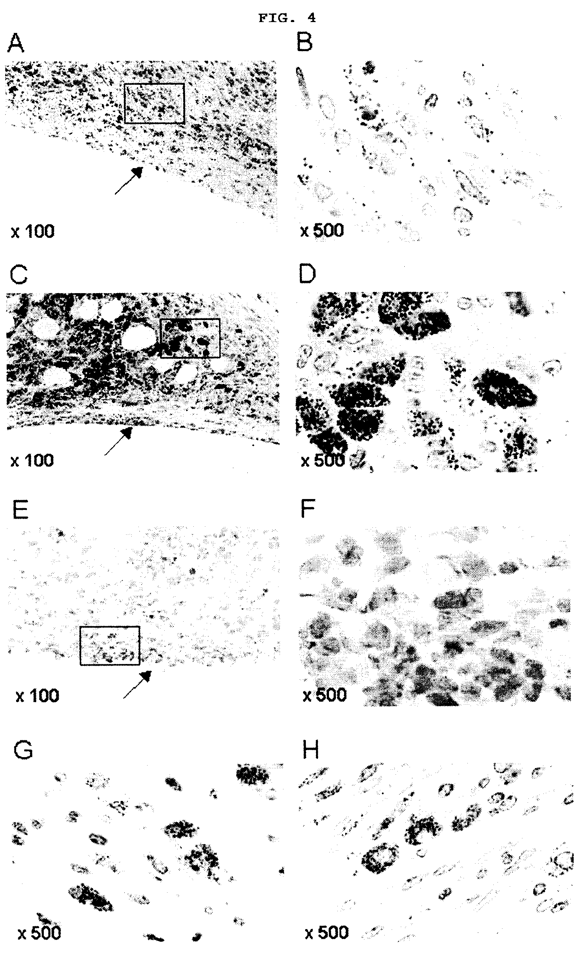

**Figure 4** shows cross sections of subcutaneously implanted catheter segments challenged with S. epidermidis, gram-stained. Tissue surrounding a PApvp catheter segment, challenged with 10⁶ cfu of S. epidermidis at 5 days, magnified x 100 (A) and x 500 (B). Tissue surrounding a PApvp catheter segment, challenged with 10⁶ cfu of S. epidermidis in septic mice, magnified x 100 (C) and x 500 (D). Figure 4 E (x 100) and F (x 500) shows that the cells containing the gram-positive cocci could be stained with the pan macrophage marker monoclonal antibody F4/80, indicating that these cells were tissue macrophages. Tissue surrounding an SEpvp catheter segment, challenged with 10⁶ cfu of S. epidermidis at 14 days, magnified x 100 (G), and tissue surrounding a SE catheter challenge with 10⁸ cfu of S. epidermidis segment at 14 days (H).

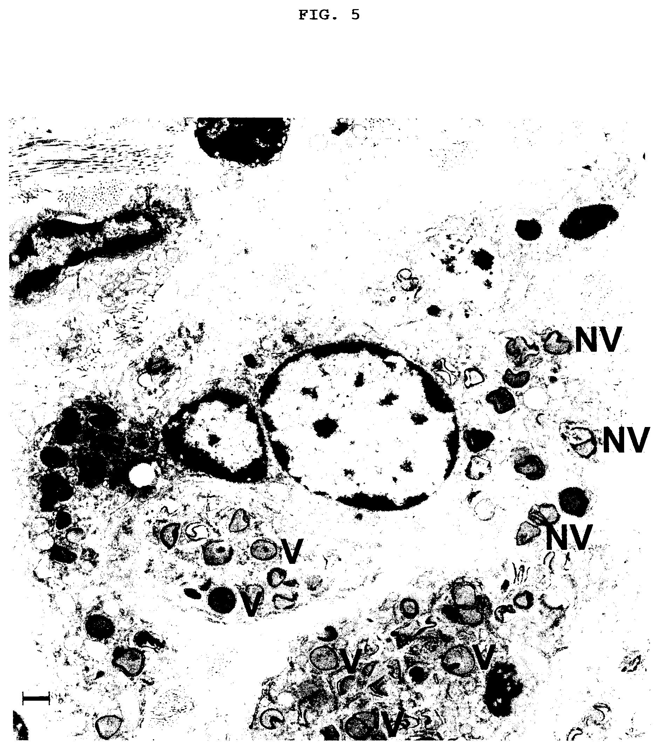

**Figure 5** shows a transmission electron microscopic photograph of macrophages in tissue surrounding PApvp 14 days after subcutaneous implantation and challenge with 10⁶ cfu S. epidermidis RP62a. The cytoplasm appears to contain numerous coccoid bacteria, part of which seem viable (V) and others exhibiting signs of degeneration indicating that they are non-viable (NV; see text). Note several bacteria having complete septa as indication for cell division (bar represents 0.5 µm).

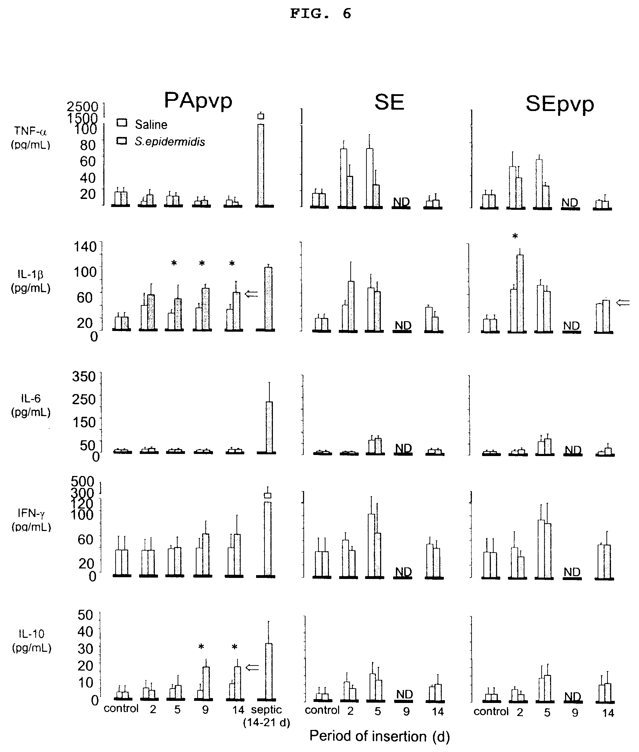

**Figure 6** shows mean (± Std.Error) insertion site concentrations of TNF-α, IL-1β, IL-6, IFN-γ and IL- 10 at various time points after subcutaneous implantation of PApvp, SE or SEpvp, challenged with saline or 10⁶ cfu S. epidermidis RP62a. (N = 4 mice per group at each time point. ^{∗}, P<0.05 for differences at indicated time point (⇒, P<0.05 for difference in over time profile between groups; ND=not determined).

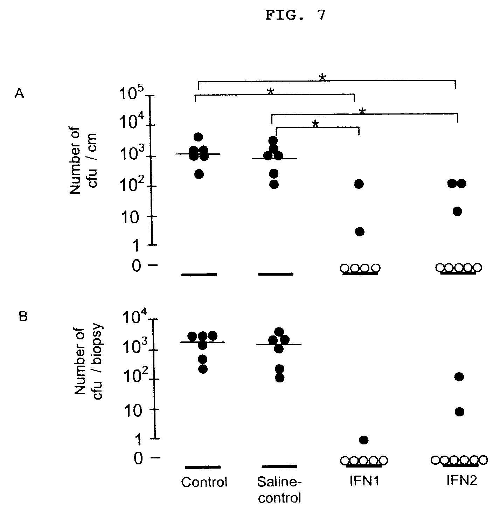

**Figure 7** demonstrates: A) Number of cfu of S. epidermidis RP62a cultured from the implantation site homogenates in the control, saline-control, IFN1 (25,000 IU 3 times weekly) and IFN2 (10,000 IU 3 times in 2 weeks) groups; B) number of adherent cfu of S. epidermidis RP62a cultured from subcutaneously implanted catheter segments in these groups 14 days after implantation and subcutaneous challenge with 10⁶ cfu of S. epidermidis RP62a. The horizontal line indicates the median; ^{∗}, P<0.05.

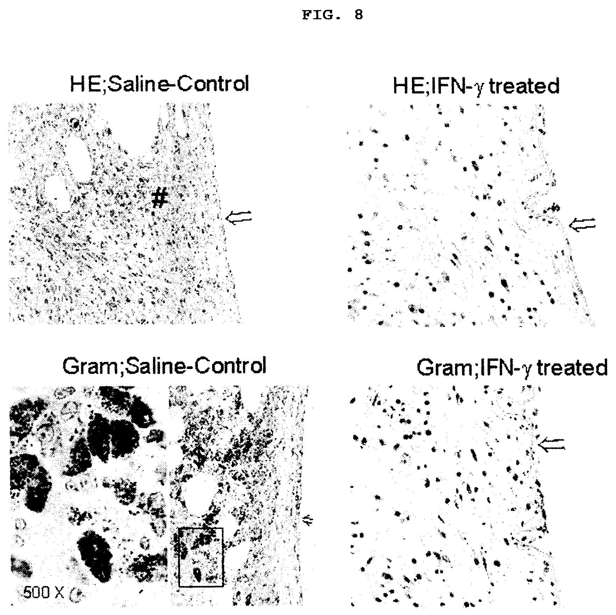

**Figure 8** shows cross sections, HE-stained and gram-stained, of subcutaneously implanted catheter segments in saline-control mice and in mice receiving 25,000 IU IFN, 3 times weekly, 14 days after subcutaneous challenge with S. epidermidis; # indicates the granuloma in tissue bordering the implant on HE-stained tissue sections; $ indicates the granuloma containing the gram-positive cocci intracellularly in macrophages on gram-stained tissue sections. The open arrows show the catheter-tissue interface. Magnification X 100.

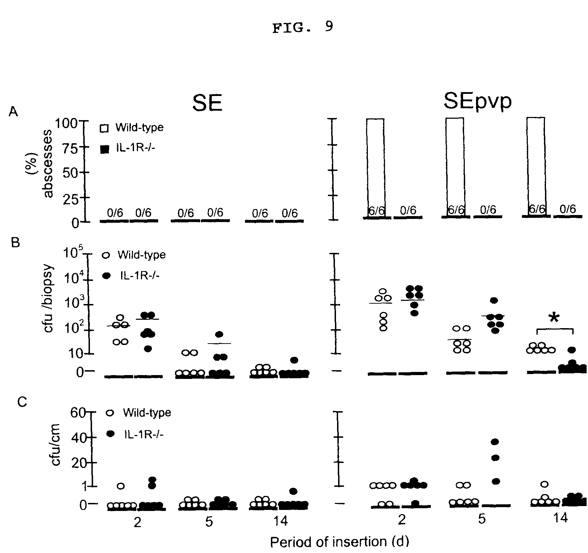

**Figure 9** shows the frequency of subcutaneous abscesses along the inserted catheter segments in wild-type mice and IL-1R-/- mice (A), number of cfu of S. epidermidis RP62a cultured from the implantation site homogenates (B) and number of adherent cfu of S. epidermidis RP62a cultured from subcutaneously implanted SE and SEpvp (C) 2 days, 5 days and 14 days after implantation and subcutaneous challenge with 10⁶ cfu of S. epidermidis RP62a (N=6 mice per group per time point; ^{∗}, P<0.05) .

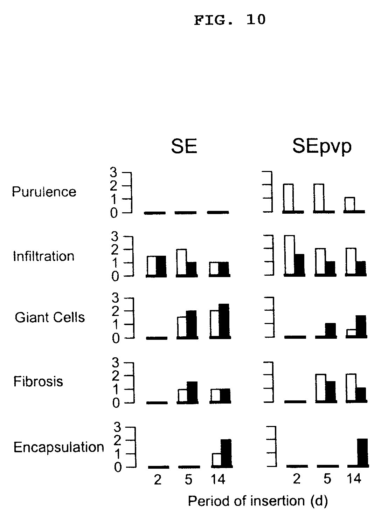

**Figure 10** shows the mean scores of purulence, infiltration, foreign body giant cells, fibrosis and encapsulation, at 2, 5 and 14 days after subcutaneous implantation of SE or SEpvp, challenged with 10⁶ cfu S. epidermidis RP62a in wild-type mice and IL-1R-/- mice (N=6 mice per group at each time point; empty bars: wild-type; filled bars: IL-1R-/-).

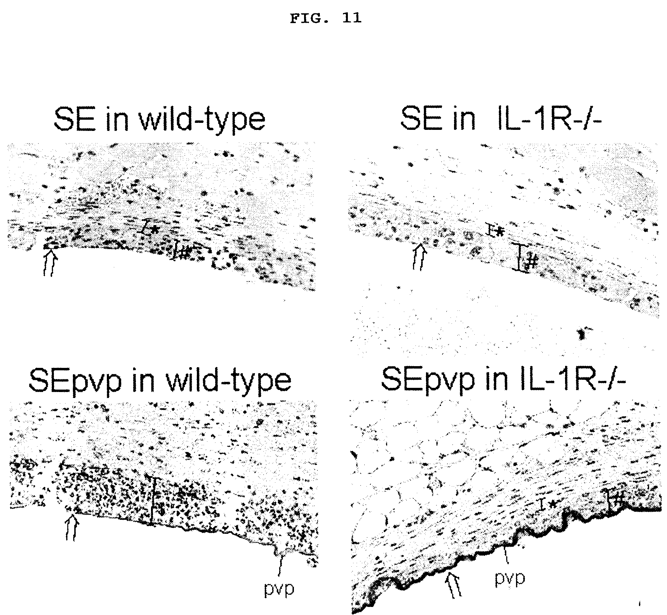

**Figure 11** shows gross sections, HE-stained, of subcutaneously implanted catheter segments in wild-type and IL-1R-/- mice 14 days after subcutaneous challenge with S.epidermidis; $ indicates abscess formation, # indicates giant cell formation, ∗ indicates the thickness of the capsule.

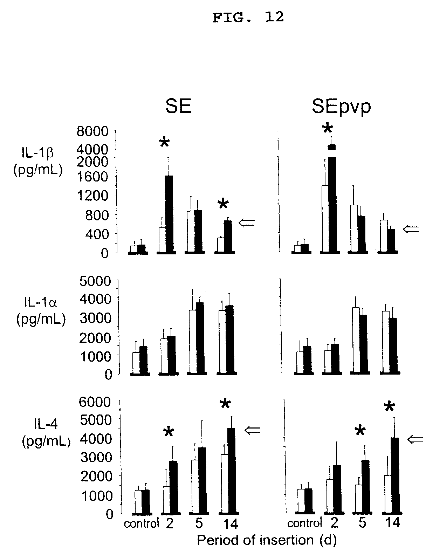

**Figure 12** demonstrates the mean (± Std.Error) implantation site concentrations of IL-1β, IL-1α and IL-4 at 2, 5 and 14 days after subcutaneous implantation of SE or SEpvp and challenge with 10⁶ cfu S. epidermidis RP62a. N=6 mice per group at each time point; ^{∗}, P<0.05 for differences between wild-type and IL-1R-/- at indicated time point; open arrow, P<0.05 for difference in over time profile between groups of wild-type and IL-1R-/- (empty bars: wild-type; filled bars: IL-1R-/-).

### EXAMPLES

### EXAMPLE 1

### Biomaterial-associated persistence of Staphylococcus epidermidis in pericatheter macrophages

In this Example the susceptibility to Staphylococcus epidermidis infection associated with 2 types of commonly used catheter materials, silicon elastomer (SE) and polyamide (PA), and their surface modified polyvinylpyrrolidone-grafted derivatives (SEpvp and PApvp, respectively) was assessed in a previously described mouse model (Christensen GD et al., J Clin Microbiol 18: 258-269, 1983). The aim of the study was to gain insight into the pathogenic process around the modified materials; (i) by localizing the niche of the surviving S. epidermidis in tissue surrounding the subcutaneously implanted catheter segments, and (ii) to determine whether specific changes in cytokine production in the pericatheter tissue are associated with the enhanced susceptibility.

### MATERIAL AND METHODS

### Animals

264 specified pathogen free female C57BL6 mice, 6-8 weeks old and weighing 15-20 g were used. All animals were housed in individual cages, in a pathogen-free environment and provided with sterile food and water.

### Catheters

Four different catheters were used, polyamide (PA), a novel catheter polyvinylpyrrolidone (pvp)-grafted polyamide (PApvp, see below), conventional silicon elastomer (SE), and pvp-grafted SE catheters (SEpvp). The PApvp catheters were manufactured at the Center for Biomaterials Research, University of Maastricht, the Netherlands. The grafting, consisting of a copolymer of N-vinylpyrrolidone and 2-(4-azidobenzoyl)-oxo-ethylmethacrylate in a molar ratio of 100:2.5 [32], was applied to thoroughly washed PA tubing with a diameter of 2.5 mm and a wall thickness of 0.6 mm by repeated dipping. Subsequently, the tubings were irradiated for 20 min with a Philips HPA 1000 high power lamp (Philips Lightning, Eindhoven, The Netherlands). The tubings were washed with propanol-2 and water (each 2 times 30 min, ultrasonic bath), dried on air and autoclaved. The SE and SEpvp catheters, which are clinically used, were obtained from Medtronic PS Medical, Goleta, CA. Segments of the catheters were cut under aseptic conditions in a laminar flow cabinet and stored in sterile petri dishes until use.

### Characterization of catheter surface characteristics

Physico-chemical characterization of PA, PApvp, SE, and SEpvp relied on two different techniques: (i), dynamic contact angle measurements according the Wilhelmy method (Andrade et al. in: Surface and interfacial aspects of biomedical polymers, first edition, Chapter 7; New York, Plenum Press, 1985); (ii), X-ray photoelectron spectroscopy (XPS).

### Preparation of Staphylococcus epidermidis inocula

S.epidermidis RP62a (ATCC 35984) was used. This strain is capable of producing slime, as determined according to Christensen et al. (Infect Immun 37: 318-326, 1982; J Clin Microbiol 22: 996-1006, 1985), and is polysaccharide/adhesin (PS/A) and polysaccharide intercellular adhesin (PIA)-positive. MIC values (µg/mL) of strain RP62a determined by standard E-test were for rifampicin <0.016, teicoplanin 0.19, gentamicin 256, minocyclin <0.016 and vancomycin 1.5. Antibiotic susceptibilities were used to confirm the identity of colonies cultured from catheter segments and tissue homogenates (see below) .

For inoculum preparation, two mL of an overnight culture of S. epidermidis strain RP62a in Trypticase Soy Broth (TSB; Difco, Detroit, MI) were inoculated into 100 mL of TSB. After incubation at 37°C for 5 h, 50 mL of the culture was centrifuged at 2200 x g for 10 min. The pelleted bacteria were washed twice with 50 mL pyrogen-free sterile isotonic saline. For in vitro adherence assays the bacteria were resuspended in TSB, and was measured. The suspension was diluted to lo⁸ cfu/mL, based on an established relationship between bacterial concentration and optical density at 620nm (A620). For mouse challenge experiments pelleted bacteria were resuspended in pyrogen-free sterile isotonic saline, the A620 measured, and the suspension diluted with pyrogen-free sterile isotonic saline to prepare the various inoculum suspensions.

### In vitro adhesion of S. epidermidis RP62a on PA, PApvp, SE and SEpvp segments

PA, PApvp, SE and SEpvp segments with a length of 10 cm were soaked in 100 mL TSB containing 10⁸ cfu/mL S. epidermidis RP62a. After 3 h of incubation at 37°C, the segments were rinsed twice with PBS. The middle 3 cm of each segment was cut out and transferred into a bottle containing 3 mL of PBS, which was sonicated (Bransonic ® B-2200 E4, 47KHz, 205 watts) for 30 s to dislodge the adherent bacteria from the segment. Numbers of cfu in the resulting suspension were assessed by quantitative culture. The sonication procedure effectively removed the adherent bacteria from the catheters as judged from scanning electron microscopic inspection of sonicated catheters, and did not affect bacterial viability.

### Subcutaneous catheter implantation

A murine model, as modified from Christensen et al (supra) was used. Mice were anaesthetized with FFM-mix (hypnorm^{®} 1 mL, midazalam^{®} 1 mL distilled water 2 mL; 0.07 mL/lOg body weight) administered intraperitoneally. Surgery was performed in a laminar flow cabinet. The back of the mice was shaved and prepared with 2% chlorohexidine in ethanol. On each side an incision (0.3 cm) was made 0.5 cm lateral to the spine. Subsequently either PA, PApvp, SE or SEpvp catheter segments with a length of 1 cm were inserted subcutaneously on both sides. Each mice received two segments of a single catheter type. The incisions were closed with a single 0/6 vicryl stitch.

### Determination of infection dose

To determine the infection dose for the four catheter materials, 5 groups of 24 mice each were used. Four experimental groups had either PA, PApvp, SE, or SEpvp catheter segments implanted. The fifth group received inoculum or saline injections only. Bacterial inocula of 10², 10⁴, 10⁶, or 10⁸ cfu in 25 µL pyrogen-free isotonic saline, or saline (25 µL) were injected subcutaneously along the inserted segments, using a repetitive pipette (Stepper, model 4001-025, Tridak division, Brookfield, CT).

### Development of infection over time

For the experiments studying bacterial persistence and the inflammatory reaction over time 144 mice were used. These mice either received PApvp, SE, or SEpvp catheter segments, were sham operated, or were left untreated to serve as inoculum controls. Mice of these 5 groups either were injected with an inoculum of 10⁶ cfu S. epidermidis in 25 µL pyrogen-free isotonic saline, or saline alone subcutaneously along the inserted segments. The sham operated mice received injections in the subcutaneous wounds, and the inoculum controls received injections in the subcutaneous tissue lateral to the spine. Mice were terminated at 2, 5, 9, 14 or 60 days after the start of the experiment. All inoculum-catheter combinations and all controls were tested in quadruplicate for each time point. In addition, 8 untreated mice were used as controls (baseline controls).

### Termination of animals and sample collection

Mice were anesthetized with FFM-mix administered intraperitoneally, terminated by cardiac puncture, and blood was collected. In a laminar flow cabinet, the skin and subcutaneous tissue were separated from the muscle fascia up to the flank on both sides. The implantation sites were inspected for inflammation, characterized by redness around the implanted segments, and for abscess formation. Subsequently, standardized biopsies of 12 mm in diameter containing skin,
subcutaneous tissue and the catheter segment were taken using a specifically developed tissue sampler. From tissue bordering the catheter segment in the right side biopsies, a smaller biopsy with a diameter of 3 mm was taken and placed in paraformaldehyde (4%), for transmission electron microscopical examination. The remainder of the 12 mm biopsies was placed in 10% buffered formaldehyde (pH 7.3).

The left side biopsies were used for quantitative culture of bacteria adherent to the catheter segment as well as bacteria residing in the tissue, and to assess cytokine levels. The catheter segments were separated from the tissue, washed twice with phosphate buffered saline (PBS; 8.1 mM Na₂HPO₄, 1.5 mM KH₂PO₄, 140 mM NaCl; pH 7.2), placed in sterile tubes containing 1 mL of PBS, and processed for quantitative culture (see below).

The tissue samples, as well as the liver, spleen, lungs and kidneys were individually placed in 6 mL tubes and weighed. The tissue samples weighed 125-160 mg, the livers 600-700 mg, the spleens 60-100 mg, the lungs 80-100 mg and the kidneys 300-350 mg. A volume of pyrogen-free isotonic saline corresponding to 4 times the weight of the specimens was added, and they were homogenised at 4°C (Tissue Tearer, model 985-370, Biospec products, Bartlesville, OK). After each homogenisation the homogeniser was carefully cleaned, disinfected with subsequent washings of 4% (w/v) sodium hypochlorite and 70% ethanol, and rinsed with pyrogen-free isotonic saline.

### Quantitative culture of PA, PApvp, SE and SEpvp segments

The tubes containing a PA, PApvp, SE or SEpvp segment in 1 mL PBS were sonicated for 30 sec in a waterbath sonicator (Bransonic ^{®}, B-2200 E4, 47KHz, 205 W) to dislodge adherent bacteria. The number of adherent S. epidermidis was assessed by quantitative culture of serial 10-fold dilutions of the PBS. Six aliquots of 10 µL of each dilution were spotted on a blood agar plate. The remaining suspension was stored at 4°C. When no growth was observed on the blood agar plates after overnight incubation at 37°C, the stored suspension was centrifuged (2200 x g, 10 min) and the pellet was resuspended in 100 µL PBS and plated on a blood agar plate. The sonicated segments were placed in 80 mL thioglycolate broth (Tb), consisting of 3% (w/v) thioglycolate, 0.03% (w/v) polyanetholesulfonic acid, 1M NaOH and 0.5% Tween 80, and incubated for 72 hours at 37°C. For statistical purposes, it was assumed that one cfu per 1 cm catheter segment had been present in case no growth occurred on the blood agar plates and the catheter segment incubated in the broth was culture-positive. The number of adherent S. epidermidis RP62a is expressed as cfu/cm catheter segment. Antibiograms were made from the cultured bacteria.

### Quantitative culture of tissue biopsy and organ homogenates, and blood

Fifty µL aliquots of the homogenates of the implantation site biopsies and of the collected organs, or 100 µL of blood, were serially 10-fold diluted with PBS. Six aliquots of 10 µL of each dilution were spotted on a blood agar plate, which was incubated overnight at 37°C. In addition, aliquots of the implantation site homogenates (50 µL), of the organ homogenates (100 µL) and of blood (50 µL) were cultured in 80 mL Tb for 72 hours at 37°C. For statistical purposes, it was assumed that one cfu per 50 µL of biopsy homogenate, per 100 µL of organ homogenate, or per 50 µL of blood had been present in case no growth occurred on the blood agar plates and the broth culture was positive. Numbers of persisting S. epidermidis RP62a are expressed as cfu/biopsy or organ, or cfu/mL of blood. Antibiograms were made from the cultured bacteria.

### Histological examination

After fixation in formaldehyde, the biopsies were embedded in methylmethacrylate / buthylmethacrylate (Merck Schuchart, Hohenbrunn, Germany), sectioned, and stained with hematoxylin-eosin. Slides were examined for 5 histological features characteristic for tissue reactions after implantation of a foreign body (Anderson JM, Trans Am Soc Artif Intern Organs 34: 101-107, 1988). These were (i) infiltration of inflammatory cells, PMNs or mononuclear cells, (ii) presence of purulence, deposition of leukocytes with necrotic burdens, (iii) foreign body giant cell formation, (iv) fibrosis, characterized by inflammatory cells, fibroblasts and newly formed collagen, and (v) encapsulation of the foreign body. Sections were scored independently by three investigators using a scale of 0 (feature absent) to 3 (feature maximally present), indicative for the grade of appearance of each feature. At the moment of judgement none of the investigators knew which sample originated from which mice.

In addition, gram-stained slides were made and inspected for gram-positive cocci in the tissue surrounding the subcutaneously inserted catheter segments. A scale, ranging from 0 to 3, indicative for the grade of presence of gram-positive cocci was made. The number of gram-positive cocci present along the entire (1 cm) segment was estimated by counting the number of gram-positive cocci in two transverse sections with a thickness of 3 µm, obtained from 2 levels in the embedded biopsy at least 1 mm apart. The mean number of bacteria in both sections was multiplied with 3333,3, in order to estimate the total number of bacteria in the tissue surrounding the 1 cm long segment. In the histological scale, grades 1 to 3 correspond to estimated numbers of 10⁴-10⁵, 10⁶-10⁷, and 10⁸-10¹⁰ of gram stained bacterial cells per biopsy, respectively.

### Transmission Electron Microscopical (TEM) examination

The 3 mm biopsies fixed in paraformaldehyde (4%) were dehydrated, and embedded in LX-112 resin according to standard methods. Areas of interest were selected on the basis of toluidine-stained light microscopic sections, and processed for ultrastructural examination.

### Macrophage immunohistochemistry

To assess whether cells containing intracellular bacteria (see results) were macrophages, some of the experiments were repeated and biopsies obtained as previously described were snap-frozen in liquid nitrogen. Immunohistochemical staining with antibody F4/80, recognizing a murine pan-macrophage marker, was performed as described previously (Laman J et al., J Neuroimmunol 86: 30-45, 1998). Briefly, 6 µm cryosections thaw-mounted on slides, were fixed with fresh acetone containing 0.02% H₂O₂ to inhibit endogenous peroxidase activity. In order to detect and to neutralize residual endogenous peroxidase activity sections were treated with 4-Cl-1-Naphthol, resulting in a dark-blue background staining. After washing with PBS/Tween, sections were incubated overnight with the primary antibody F4/80 (rat anti-mouse), followed by rabbit anti-rat peroxidase labeled IgG (DAKO, Glostrup, Danmark). 3-Amino-9-ethylcarbazole (AEC: Sigma, St. Louis, MO) was used to detect the peroxidase activity of this conjugate, resulting in a bright red precipitate. Sections were counter-stained with haematoxilin for 10 seconds and mounted in Kaiser's glycerin-gelatin.

### Cytokine assays of the implantation site tissue homogenates

The implantation site biopsy homogenates, reduced by 100 µL which was used for quantitative culture, were diluted in 1 volume lysis buffer containing 1% Triton X-100, 150 mM NaCl, 30 mM Tris HCl, 2 mM CaCl₂, 2 mM MgCl₂, 0.2% aprotin and 0.2% leupeptin, pH 7.4 (Greenberger M, et al., J Immunol 155: 722-729, 1995), and incubated on ice for 1 h. Subsequently, the homogenates were centrifuged at 130,000 x g for 15 min at 4°C to remove cell debris. The cell-free supernatants were frozen at -80°C, thawed, centrifuged at 5000 x g to remove macro-aggregates, and stored in aliquots of 35 µL at -80°C until use. Interleukin (IL)-1β (R&D systems, Minneapolis, MN), IL-6 (Pharmingen, San Diego, CA), IL-10 (R&D systems), Tumor necrosis factor (TNF)-α (Pharmingen) and interferon (IFN)-γ (R&D systems) were measured using commercially available ELISA-kits. Cytokine levels are expressed as picograms (pg) per mL homogenate.

### Statistical analysis

All values are expressed as Mean ± Std.Error. Two sample comparisons were made by analysis of variance (ANOVA) and comparisons between over time cytokine levels of groups by ANOVA in a general linear model - general factorial. P<0.05 was considered significant.

### RESULTS

### Surface characteristics of PA, PApvp, SE and SEpvp catheters

The contact angle measurements yielded a receding contact angle of 45° ± 6° for PA, and 24° ± 5° for PApvp. The receding contact angles for SE and SEpvp were 38° ± 4° and 24° ± 4°, respectively. These data show that a hydrophilic coating is present on PApvp and SEpvp.

The XPS spectra clearly revealed the purity of the four materials. The spectra of PA and PApvp were highly similar. Pvp is composed of nitrogen, carbon, and hydrogen, and these elements are also present in PA. In addition, the pvp grafting most likely is thinner than the surface layer of PA measured by XPS. Angle-resolved XPS, by which a thinner surface layer can be analyzed, could not be used since this method is only feasible for flat surfaces and the catheter materials of the present study were cylindrical. The XPS results however do not exclude the presence of a thin pvp grafting at the surface of PApvp. The XPS spectra of SE and SEpvp differed as follows. An Nls line was characteristic for the SEpvp spectrum (399.8 eV), indicating the presence of a pvp grafting. Nitrogen signals were absent in the SE spectrum.

### Adhesion of S. epidermidis RP62a on PA, PApvp, SE and SEpvp segments in vitro

The catheter materials grafted with the pvp hydrogel in vitro had significantly lower numbers of adherent bacteria than the non-grafted catheters. The pvp grafting reduced the bacterial adherence from 16,111 ± 6,123 cfu per cm on PA to 2,188 ± 512 cfu per cm on PApvp catheter segments (P<0.001), and from 21,121 ± 2,000 cfu per cm on SE to 2,468 ± 312 cfu per cm on SEpvp catheter segments (P<0.001).

### Inoculum size and infection associated with subcutaneously inserted PA, PApvp, SE and SEpvp

The infection dose of S. epidermidis for PA, PApvp, SE, and SEpvp was assessed by challenging mice with 10², 10⁴, 10⁶ and 10⁸ cfu. At 14 days after challenge no purulence or other signs of inflammation were observed macroscopically around PA, PApvp and SE segments. In contrast, around subcutaneously inserted SEpvp segments challenged with bacterial inocula of 10⁴ cfu or higher, purulence had developed along the inserted catheter (figure 1). Mice carrying PA, PApvp, SE or SEpvp segments having received a saline injection, did not develop signs of inflammation around the segments. Subcutaneous injection of the various bacterial inocula in mice without implanted segments did not induce purulence or other signs of inflammation either (not shown).

Large numbers of S. epidermidis (3500 cfu/biopsy) were cultured from tissue homogenates of PApvp implantation sites challenged with 10⁶ or 10⁸ cfu. From the corresponding PApvp segments, mean numbers of 900 and 800 cfu/cm were cultured, respectively (figure 1). Conversely, only the PA pericatheter tissue of mice challenged with 10⁸ cfu was culture positive, and only 2 of the 4 explanted segments yielded growth of low numbers of S. epidermidis. Homogenates of tissue from SE implantation sites as well as the explanted segments were culture negative, except for one of tissue challenged with 10⁸ cfu (figure 1). Homogenates of SEpvp pericatheter tissue challenged with 10⁶ or 10⁸ cfu S. epidermidis contained low numbers of bacteria, since only broth cultures were positive (figure 1). The explanted segments were all culture negative. No S. epidermidis was cultured from the homogenates of any of the mice which only received an injection of the various inocula (not shown). In all cases bacteria cultured from tissue or catheter segments had the same antibiogram as S. epidermidis RP62a.

### Development of infection over time

Subsequently, the persistence of S. epidermidis around PApvp, SE and SEpvp in mice challenged with 10⁶ cfu over time up to 60 days was studied. Since the bacteriological and histological findings (not shown) from PA and SE were very similar, and the SE catheter is clinically used, the PA catheter material was excluded from these subsequent experiments. Around PApvp and SE, macroscopically no purulence or other signs of inflammation were seen at any time point. Around SEpvp segments purulence was present at all time points (figure 2). At 60 days purulence was observed only inside the SEpvp catheter segment and not around the inserted segment.

At 2 days significantly more S. epidermidis (P<0.001) was cultured from the tissue homogenates of PApvp and SEpvp implantation sites (27,300 ± 24,000 and 17,000 ± 1,400, respectively) than from those of SE implantation sites (1,100 ± 400; figure 2). From all PApvp and SEpvp segments adherent bacteria were cultured. The quantitative cultures of SE segments were all negative, and the broth culture of only 1 segment was positive (figure 2).

At 5, 9, and 14 days all tissue homogenates of PApvp implantation sites were culture positive. The mean numbers of cfu per biopsy ranged from 260 at 5 days up to 3,500 at 14 days (figure 2). Three of the 4 S. epidermidis-challenged mice with subcutaneously implanted PApvp, which were to be terminated at 60 days, developed sepsis between 14 and 21 days. These mice were terminated and were evaluated as a separate group ("septic mice"). They had mean numbers of cfu per biopsy ranging from 2,352 to 3,567. The fourth mouse of this group was terminated after 60 days, and 900 cfu were cultured from the biopsy of this mouse. From 14 of the 16 subcutaneously inserted PApvp segments removed from day 5 to 60 adherent S. epidermidis were cultured,_ranging from 260 to 800 cfu/cm (days 5 and 14, respectively; figure 2).

At 5, 9, 14, and 60 days the homogenates of the SE pericatheter tissue, as well as the explanted segments were all culture negative. Of the 12 SEpvp pericatheter tissue homogenates of 5, 14 and 60 days, only the broth cultures were positive. At 5 days, the broth cultures of 2 out of 4 SEpvp segments were positive (figure 2). All SEpvp segments removed at 14 and 60 days were culture-negative. In all cases, the bacteria cultured from tissues or segments had the same antibiogram as S. epidermidis RP62a.

Thus, around PApvp no abscesses were observed, but high numbers of bacteria persisted on the catheter segment and in the pericatheter tissue. On and around SE almost no persisting S. epidermidis were found, not even at the highest inoculum dose. Around SEpvp abscesses were induced and low numbers of bacteria persisted in the pericatheter tissue, whereas no adherent bacteria could be cultured at the later time points (5 - 60 days).

### Dissemination of S. epidermidis to blood and organs

Blood and organ cultures from mice with implanted SE or SEpvp segments showed no growth. Although the quantitative cultures of blood or organ homogenates from mice with PApvp segments were negative, qualitative blood cultures were positive in 4 of the 20 mice, one terminated at 9 days, one at 14 days and two in the "septic mice" group. The broth cultures of the spleen from 3 of these 4 mice, one terminated at 9 days, one at 14 days and one in the "septic mice" group, were also positive. The bacteria cultured had the same antibiogram as S. epidermidis RP62a. Apparently, S. epidermidis RP62a persisting in the pericatheter tissue around PApvp were capable of causing bacteremia.

### Histological examination of the inflammatory and foreign body response over time

Five histological features characteristic for the tissue inflammatory response following the implantation of a foreign body were scored independently and blindly by 3 investigators. The mean scores for four of the five histological features at each time point are shown in figure 3. Variation of scores between investigators was always maximally 0.5 scale units.

Up to 14 days the foreign body inflammatory response around PApvp and SE, characterized by the grade of infiltration, fibrosis, foreign body giant cell (FBGC) formation and encapsulation was similar, irrespective of the presence of S. epidermidis (figure 3). In mice with implanted PApvp which became septic, FBGC formation was increased compared to that in mice terminated at 14 days. In contrast, around SE, FBGC formation had decreased to a low level at 60 days. Around SEpvp the inflammatory response was stronger than around PApvp and SE at all time points. Infiltration and fibrosis around SEpvp were scored higher than around PApvp and SE at all time points, and FBGC formation and encapsulation around SEpvp were delayed, irrespective of the presence of S. epidermidis. These findings indicate that an apparently normal foreign body reaction follows implantation of SE, and that tissue around SEpvp had a prolonged and intensified inflammatory status. Around PApvp a normal foreign body reaction was seen up to 14 days, irrespective of the presence of S. epidermidis. In presence of S. epidermidis, however, a high FBGC score was found in septic mice, while around sterile PApvp the score decreased after 14 days. It should be noted that the infiltration observed in the pericatheter tissue at 2 days consisted of a mixed population of PMNs and macrophages, while at 5 days and later the infiltration predominantly consisted of macrophages for around either catheter material.

### Intracellular presence of S. epidermidis in macrophages

In gram-stained tissue sections of mice with subcutaneously inserted PApvp challenged with 10⁶ cfu of S. epidermidis, large numbers of gram-positive cocci were seen (figure 4A-D). Almost all gram-stained cocci were located intracellularly and their numbers increased over time. In tissue of mice terminated at 5 days less infected cells and less cocci per cell were present (figure 4A,B) in comparison to tissue section from septic mice (figure 4C,D) .

The cells containing the gram-positive cocci were stained with the pan-macrophage marker monoclonal antibody F4/80, indicating that these cells were tissue macrophages (figure 4E,F). The number of gram-positive cocci observed in tissue surrounding PApvp increased over time. In some macrophages several hundreds of gram-stained cocci were observed within the thin section (figure 4C,D). As the histology suggested that much more bacteria were present in tissue surrounding the PApvp than were cultured from the corresponding tissue homogenate, we estimated the number of bacteria present in tissue surrounding PApvp, based on counts in the microscopic slides. The estimated number of S. epidermidis increased from 10⁵ cocci per biopsy on 2 and 5 days, to values of 10¹⁰ individual cocci per biopsy in mice which became septic after 14 - 21 days (figure 4A-D; table 1). Around SEpvp low levels of intracellular bacterial persistence in macrophages were seen at 5, 14 and 60 days (figure 4G). All broth cultures at these time-points were positive. In contrast, in sections of tissue around SE and PA challenged with 10⁶ cfu, no gram-positive cocci were seen and the corresponding homogenates were culture negative. However, in sections obtained from samples of tissue around SE injected with 10⁸ cfu, intracellular cocci were observed (figure 4H), and one of the 4 quantitative cultures of tissue surrounding SE was positive. Also in the pericatheter tissue around PA challenged with 10⁸ cfu, intracellular cocci were observed (not shown). The tissue cultures were positive.

### Electron microscopy

Coccoid bacteria were located in vacuoles within the macrophages, and showed different morphologies (figure 5). Some bacteria appeared in rounded structures, about 0.5 µm in diameter and consisting of a single cell-wall with moderately electron-dense contents, apparently representing viable bacteria. Apparently non-viable bacteria were also observed, as was evident from the presence of various signs of degeneration, like loss of the spherical shape, fragmentation of the cell wall, and rarefaction of the contents. In the bacteria that appeared viable at the time of fixation, the cell wall had often formed a complete or a partial septum, indicating cell division.

**Table 1**

| Score for presence of S. epidermidis in tissue surrounding the 3 biomaterials | | | |
|---|---|---|---|
| Time point (days) | Material | | |
| | PApvp | SE | SEpvp |
| 2 | 1.5 | 0 | 0 |
| 5 | 1.5 | 0 | 0 |
| 9 | 2 | 0 | 0.5 |
| 14 | 2 | 0 | 0.5 |
| septic | 3 (n=3) | | |
| 60 | 1.5 (n=1) | 0 | 0.5 |

### Induction of TNF-α, IL-1β, IL-6, IFN-γ and IL-10

In mice with subcutaneously implanted SE or SEpvp catheter segments, over time TNF-α, IL-1β, IL-6 and IFN-γ tissue levels were elevated compared to sham and baseline controls. Only levels of IL-1β differed between SE and SEpvp. Levels of IL-1β around sterile SEpvp were elevated earlier (t = 2d) and remained higher up to 14 days than around sterile SE. In tissue around SEpvp challenged with S. epidermidis, the IL-1β profiles were significantly higher than around SE challenged with S. epidermidis. It was concluded that the abscess formation around SEpvp challenged with S. epidermidis was associated with an enhanced IL-1β production resulting in a protracted inflammatory status of the tissue.

In tissue surrounding PApvp injected with saline, no increase in the levels of TNF-α, IL-1β, IL-6, IFN-γ or IL-10 was found (figure 6) in comparison to baseline control levels. When S. epidermidis was injected along the subcutaneously inserted PApvp segments, TNF-α, IL-6 and IFN-γ levels were not increased either. Local IL-1β production over time was significantly (P<0.05) increased in comparison to the levels in the baseline controls and PApvp implant controls (figure 6). This indicates that the intracellular survival of S. epidermidis in macrophages is associated with IL-1β production in tissue surrounding PApvp. In tissue around SE and SEpvp either injected with saline or S. epidermidis the IFN-γ levels were increased at day 5 compared to those at day 2, and to those of the baseline controls. In tissue around PApvp such an IFN-γ increase was not observed. Local IL-10 production was not upregulated at day 2 and day 5, but from day 9 levels were significantly higher than the baseline control levels (P=0.018). In septic mice, TNF-α production was 100-fold higher than levels in control mice, as well as the levels of the other cytokines tested (P=0.000; figure 6). The sham and injection control groups did not show elevated levels of any of the tested cytokines in comparison with the baseline control levels (not shown).

In summary, abscesses developed around SEpvp, whereas around SE, PA and PApvp no signs of infection were observed, although mice carrying PApvp developed septicemia after 14 - 21 days. S. epidermidis was cultured from the tissue surrounding PApvp. Cells around PApvp, containing large numbers of bacteria, were identified as macrophages using immunohistochemistry and electron microscopy. The persistence of intracellular bacteria was also observed around SEpvp, SE and PA, albeit to a lesser extent. It was demonstrated that implanted biomaterial induces an inflammatory response favorable to the persistence of S. epidermidis.

### CONCLUSION

In conclusion, this Example is the first to describe the intracellular persistence of bacteria in macrophages as a possible cause of biomaterial-associated infection.

### EXAMPLE 2

### Interferon-y protects against biomaterial-associated S. epidermidis infection in mice

In the present Example it was tested whether subcutaneous administration of IFN-γ could reduce the susceptibility to infection in a mouse model of biomaterial-associated S. epidermidis infection (BAI).

### MATERIAL AND METHODS

### Animals

A total of 26 specified pathogen free female C57BL6 mice, 6-8 weeks old and weighing 15-20 g were used. All animals were housed in individual cages, in a pathogen-free environment and provided with sterile food and water.

### Catheter segments

Catheters of polyvinylpyrrolidone-coated polyamide with a diameter of 2.5 mm and a wall thickness of 0.6 mm were manufactured at the Center for Biomaterials Research, University of Maastricht, the Netherlands. One cm long segments, cut from either catheter under aseptic conditions in a laminar flow cabinet were stored in sterile petri dishes.

### Preparation of Staphylococcus epidermidis inocula

The slime producing S. epidermidis RP62a (ATCC 35984) strain was used as the challenge organism. MIC values (µg/mL) of strain RP62a determined by standard E-test were for rifampicin <0.016, teicoplanin 0.19, gentamicin 256, minocyclin <0.016 and vancomycin 1.5. Antibiotic susceptibilities were used to confirm the identity of colonies cultured from catheter segments and tissue homogenates (see below). An inoculum of 10⁶ cfu / 25 µL was prepared by growing S. epidermidis to midlogarithmic phase at 37°C in Trypticase Soy Broth (TSB; Difco, Detroit, MI) and diluting the suspension with pyrogen-free isotonic saline, based on an established relationship between bacterial concentration and optical density at 620 nm. The injection of this inoculum along implanted catheter segments reproducibly induced a BAI in this mouse model, as described in Example 1.

### Induction of a biomaterial-associated infection in mice

Mice were anesthetized with FFM-mix (hypnorm^{®} 1 mL, midazalam^{®} 1 mL distilled water 2 mL; 0.07 mL/lOg body weight) administered intraperitoneally. Surgery was performed in a laminar flow cabinet. The back of the mice was shaved and prepared with 2% chlorhexidine. On each side an incision (0.3 cm) was made 0.5 cm lateral to the spine. Subsequently catheter segments with a length of 1 cm were inserted subcutaneously on both sides. The incisions were closed with a single 0/6 vicryl stitch. Subsequently, the bacterial inoculum was injected subcutaneously along the inserted segments.

### Subcutaneous administration of IFN-γ

Mice were divided into four groups. The first group (IFN1; n=6) received subcutaneous injections of 25µL pyrogen-free isotonic saline containing 25,000 IU carrier-free recombinant murine-IFN-γ (rmIFN-γ; R&D systems, Minneapolis, MN), 3 times weekly, the second group (IFN2; n=8), 10,000 IU rmIFN-γ, 3 times in 2 weeks, the third group (saline-control; n=6) saline only, 3 times weekly, and the fourth group (control; n=6), did not receive any injection.

### Sample collection

Fourteen days after implantation mice were anaesthetised with FFM-mix, and terminated by cardiac puncture. Terminated mice were placed in a laminar flow cabinet and fixed on a sterile polypropylene plate. A dorsal midline incision was made from the cervical to the lumbal area. Subsequently, the skin and subcutaneous tissue were separated from the muscle fascia, up to the flank on both sides. The implantation sites were inspected for purulence and standardized biopsies (φ 12mm) were taken from the implantation sites using a specially developed tissue sampler. Each single biopsy included skin, subcutaneous tissue and the inserted segment.

The right side biopsy from each mouse was placed in 10% buffered formaldehyde (pH 7.3) for histological examination. From the left side biopsy, the catheter segment was separated from the tissue, washed twice with phosphate buffered saline (PBS) and placed in a sterile tube containing 1 mL of PBS. Each tissue sample was placed in a tube, weighed, and a volume of pyrogen-free isotonic saline corresponding to 4 times the weight was added. The weight of the tissue samples varied between 125 and 160 mg.

### Histological examination

After fixation in formaldehyde, the biopsies were embedded in plastic, methylmethacrylaat / butylmethacrylaat (Merck Schuchart, Hohenbrunn, Germany), sectioned, and stained with hematoxylin-eosin (HE).

Slides were examined for 5 histological features characteristic for tissue reactions after implantation of a foreign body (Anderson et al., Trans Am Soc Artif Intern Organs 34: 101-107, 1988). These were (i) infiltration of inflammatory cells, polymorphonuclear (PMN) or mononuclear cells, (ii) presence of purulence, deposition of leukocytes with necrotic burdens, (iii) foreign body giant cell formation, (iv) fibrosis, characterized by inflammatory cells, fibroblast and newly formed collagen and (v) encapsulation of the foreign body. In addition, gram-stained slides of tissue sections were made.

### Quantitative culturing of the catheter segments and tissue homogenates

The tubes containing the segments in 1 mL PBS were sonicated for 30 sec in a waterbath sonicator (Bransonic ^{®}, B-2200 E4, 47KHz, 205 W) to dislodge adherent bacteria. Tissue samples in pyrogen-free isotonic saline were homogenized on ice with a tissue homogenizer (Tissue Tearer, model 985-370, Biospec products, Bartlesville, OK). The suspensions in both tubes were quantitatively cultured, overnight at 37°C, to determine the number of cfu of S. epidermidis adherent on the segment and the number of cfu residing in the tissue. In addition, 50 µL of the undiluted suspension as well as the catheter segment were incubated in 80 mL thioglycolate broth consisting of 3% (w/v) thioglycolate containing 0.03% (w/v) polyanetholesulfonic acid, 1M NaOH and 0.5% Tween 80, for 72 hours at 37°C. For statistical purposes, it was assumed that one cfu per 1 cm catheter segment or 1 cfu per biopsy had been present in case no growth occurred on the blood agar plates, while the catheter segment or the aliquot of the tissue homogenate incubated in the broth was culture-positive. The number of adherent S. epidermidis RP62a is expressed as cfu/catheter segment (1 cm) and the number of persisting S. epidermidis RP62a in tissue as cfu/biopsy.

### Data analysis

All values are expressed as Mean ± Std.Error. Two sample comparisons were made by analysis of variance (ANOVA). The significance of differences between the frequencies of categorical variables was determined using the χ-square or Fisher's exact tests. P≤ 0.05 was considered to be statistically significant.

### RESULTS

### Infection associated with subcutaneously implanted catheter segment

At 14 days after challenge all mice were killed. No purulence or signs of inflammation were observed macroscopically around any of the catheter segments in any group. In the control and saline-control groups, all 6 segments (773±1,046 and 805±1,051 cfu/cm, respectively), and all 6 tissue homogenates (3,513±1,514 and 2,552±1,771 cfu/biopsy, respectively), were culture-positive for S. epidermidis (figure 7). In contrast, in the IFN1 and IFN2 groups significantly less segments (2 out of 6 and 3 out of 8, respectively; both P<0.05 vs. either control group), and significantly less tissue homogenates were culture-positive (1 out of 6 and 2 out of 8, respectively; both P<0.05 vs. either control group; figure 7). In the 3 out of 8 segments that were culture positive in the IFN2 group, the number of cultured cfu (121±80) was significantly lower (P<0.01) than in the non-injected and saline-control-groups (figure 7). All cultured colonies had the same antibiogram as S. epidermidis RP62a. Hence, mice treated with IFN-γ were less susceptible to BAI due to S. epidermidis.

### Histological examination of the inflammatory and foreign body response

Around the catheter segments in the control and saline-control groups a moderate mononuclear inflammation and fibrosis was observed. In addition, a thick layer containing giant cells bordering the catheter, associated with a granuloma was observed (figure 8). In contrast, in the IFN1 and IFN2 groups no layer containing giant cells bordering the catheter-tissue interface, nor an associated granuloma was observed. The tissue surrounding the catheter segments in the IFN1 and IFN2 group was moderately inflamed with mainly mononuclear cells.

### Intracellular persistence of S. epidermidis in macrophages

In the control and saline-control groups large numbers of macrophages containing gram-positive cocci were observed in the granulomatous tissue surrounding the implanted catheter segment (figure 8). In contrast, in the IFN1 and IFN2 group neither intracellular persistence nor extracellular persistence of gram-positive cocci was observed around implanted segments (figure 8).

### CONCLUSION

In summary, in the present Example it has been found that mice injected with either a high or low dose of IFN-y are less susceptible to BAI. The abundant amounts of intracellularly persisting gram-positive cocci observed in the control mice were absent in the IFN-γ treated mice. It has thus been demonstrated that subcutaneous injections of IFN-γ prevented intracellular persistence of S. epidermidis in the vicinity of an implanted biomaterial in mice.

### EXAMPLE 3

### Interleukin-1 receptor type 1 gene-deficient mice are less susceptible to Staphylococcus epidermidis biomaterial-associated infection

IL-1α and IL-1β are potent pro-inflammatory cytokines that have been implicated as important mediators in the pathogenesis of a variety of immunological and infectious diseases. IL-1 exerts biological effects by an interaction with the type I IL-1 receptor (IL-1R). In the present Example, the susceptibility of IL-1R gene deficient (IL-1R-/-) mice to biomaterial-associated infections caused by S. epidermidis was evaluated.

### MATERIAL AND METHODS

### Animals

A total of 80 specified pathogen free IL-1R -/-mice, back-crossed six times to C57BL/6 background, and C57BL/6 wild-type mice, 6-8 weeks old and weighing 15-20 g, were used. IL-1R -/- mice (Glaccum M et al., J Immunol 159: 3364-3371, 1997) were kindly provided by Immunex Co (Seattle, WA) and bred in the animal facility. Wild-type C57BL/6 mice were from Harlan, Horst, the Netherlands. IL-1R -/- and wild-type mice were age-, weight-, and sex-matched, housed in individual cages in a pathogen free environment and provided with sterile food and water.

### Catheter segments

Catheters of conventional silicone elastomer (SE) and polyvinylpyrrolidone-grafted silicone elastomer (SEpvp; Bioglide TM), with a diameter of 2.5 mm and a wall thickness of 0.6 mm were obtained from Medtronic PS Medical, Goleta, CA. One cm long segments of SE and SEpvp, cut from either catheter under aseptic conditions in a laminar flow cabinet were stored in sterile petri dishes.

### Bacterial strain

The clinical isolate Staphylococcus epidermidis strain RP62a (ATCC 35984) was used. This strain is capable of producing slime, determined according to Christensen et al. (supra) . MIC values (µg/ml) of strain RP62a determined by standard E-test, were for rifampicin <0.016, teicoplanin 0.19, gentamicin 256, minocyclin <0.016 and vancomycin 1.5. Antibiotic susceptibilities were used to confirm the identity of bacteria cultured from catheter segments and tissue homogenates.

### Preparation of the bacterial inoculum

An inoculum of 10⁶ cfu of S. epidermidis RP62a was used. It has previously been demonstrated that in mice injected with this inoculum along subcutaneously implanted SEpvp, macroscopic abscesses formation and persistent infection was induced, while around SE neither abscess formation nor infection were observed. Two ml of an overnight culture of strain RP62a in Trypticase Soy Broth (TSB; Difco, Detroit, MI) was inoculated into 100 ml fresh TSB. After incubation at 37°C for 5 hours, 50 ml of this culture were centrifuged at 2200 x g for 10 min, and the pelleted bacteria were washed twice with 50 ml pyrogen-free isotonic saline. After the final centrifugation, the pellet was resuspended in pyrogen-free isotonic saline and optical density at 620 nm was measured. The inoculum of 10⁶ cfu was prepared by growing S. epidermidis to midlogarithmic phase at 37°C in Trypticase Soy Broth (TSB; Difco, Detroit, MI) and diluting the suspension with pyrogen-free isotonic saline, based on an established relationship between bacterial concentration and optical density at 620 nm. The injection of this inoculum along implanted catheter segments reproducibly induced a BAI in this mouse model, as described in Example 1.

### Subcutaneous catheter implantation and administration of the bacterial inoculum

Mice were anesthetized with an FFM-mix (1 mL of hypnorm^{®}, 1 mL of midazalam^{®} and 2 mL of distilled water; 0.07 mL/l0g body weight), administered intraperitoneally, and placed in a laminar flow cabinet. The back of the mice was shaved and prepared with 2% (w/v) chlorhexidine. On each side an incision (0.3 cm) was made 0.5 cm lateral to the spine. Subsequently, 1 cm long SE or SEpvp catheter segments were inserted subcutaneously. IL-1R -/- and wild-type mice received either 2 SE or 2 SEpvp segments. The incisions were closed with a single 0/6 vicryl stitch. Mice with implanted SE or SEpvp segments were injected with the inoculum subcutaneously along the inserted segments in a 25µL volume using a repetitive pipette (Stepper, model 4001-025, Tridak division, Brookfield, CT). Four IL-1R -/- mice and 4 wild-type mice received a subcutaneous injection of saline only, on both sides of the spine (saline controls).

### Sample collection

Mice in the saline control group were sacrificed and evaluated 14 days after injection. After 2, 5 or 14 days, six mice of each of the experimental groups were anaesthetised with an FFM-mix administered intraperitoneally, and subsequently terminated by cardiac puncture. Terminated mice were placed in a laminar flow cabinet and fixed on a sterile polypropylene plate. A dorsal midline incision was made from the cervical to the lumbal area. Subsequently, the skin and subcutaneous tissue were separated from the muscle fascia, up to the flank on both sides. The implantation sites were inspected for purulence and standardised biopsies (φ 12mm) were taken from the implantation sites using a specially developed tissue sampler. Each single biopsy included skin, subcutaneous tissue and the inserted segment.

The right side biopsy from each mouse was placed in 10% buffered formaldehyde (pH 7.3) for histological examination. From the left side biopsy, the catheter segment was separated from the tissue, washed twice with phosphate buffered saline (PBS; 8.1 mM Na₂HPO₄, 1.5 mM KH₂PO₄, 140 mM NaCl; pH 7.2) and placed in a sterile tube containing 1 mL of PBS. The tissue sample was placed in a tube, weighed, and a volume of pyrogen-free isotonic saline corresponding to 4 times the weight was added. The weight of the tissue samples varied between 125 and 160 mg.

### Histological examination

After fixation in formaldehyde, the biopsies were embedded in plastic, methylmethacrylaat / butylmethacrylaat (Merck Schuchart, Hohenbrunn, Germany), sectioned, and stained with hematoxylin-eosin (HE). Slides were examined for 5 histological features characteristic for tissue reactions after implantation of a foreign body (Anderson et al., supra). These were (i) infiltration of inflammatory cells, polymorphonuclear (PMN) or mononuclear cells, (ii) presence of purulence, deposition of leukocytes with necrotic burdens, (iii) foreign body giant cell formation, (iv) fibrosis, characterized by inflammatory cells, fibroblast and newly formed collagen and (v) encapsulation of the foreign body. Sections were scored independently by three investigators, using a scale of 0 to 3, indicative for the grade of appearance of each feature. A feature was scored "0", when it was not observed; a feature was scored "3" when it was maximally present. None of the investigators knew which sample originated from which mouse. The scales were based on inspection of slides previously obtained from tissue samples taken from subcutaneously inserted SE or SEpvp segments in mice which received bacterial inocula ranging from 0 (saline injection) to 10¹⁰ cfu of S. epidermidis RP62a, along the inserts.

### Quantitative culture of SE and SEpvp catheter segments

The tubes containing SE and SEpvp segments in 1 mL PBS were sonicated for 30 sec in a waterbath sonicator (Bransonic ^{®}, B-2200 E4, 47KHz, 205 W) to dislogde adherent bacteria. The number of viable S. epidermidis was assessed by quantitative culture of serial 10-fold dilutions of this PBS sonicate. Six aliquots of 10 µL of each dilution were spotted on a blood agar plate which was incubated overnight at 37°C. The remaining suspension was stored at 4°C. When no growth was observed on the blood agar plates, the stored suspension was centrifuged (2200 x g, 10min) and the pellet was resuspended in 100 µL PBS and plated on blood agar. In addition, the sonicated segments were cultured in 80 ml modified thioglycolate broth consisting of 3% (w/v) thioglycolate containing 0.03% (w/v) polyanetholesulfonic acid, 1M NaOH and 0.5% Tween 80, for 72 hours at 37°C. For statistical purposes, it was assumed that one cfu per 1 cm catheter segment had been present in case no growth occurred on the blood agar plates and the catheter segment incubated in the broth was culture-positive. The number of adherent S. epidermidis RP62a is expressed as number of cfu/catheter segment (1 cm).

### Quantitative culture of the homogenates

Tubes containing the tissue sample in pyrogen-free isotonic saline were homogenized on ice with a tissue homogenizer (Tissue Tearer, model 985-370, Biospec products, Bartlesville, OK). After each homogenization the homogenizer was carefully cleaned, disinfected by subsequently washing in 4% (w/v) sodium hypochloride, 70% alcohol, and rinsed with pyrogen-free isotonic saline. Seventy µL of the homogenates was serially 10-fold diluted and 6 .aliquots of 10 µL of undiluted homogenate and of each dilution were spotted on a blood agar plate, which was incubated overnight at 37°C. In addition, 50 µL of the homogenate was cultured in 80 ml thioglycolate broth for 72 hours at 37°C. For statistical purposes it was assumed that one cfu per 50 µL had been present in case no growth occurred on the blood agar plates and the homogenate aliquot incubated in the broth was culture-positive. The number of persisting S. epidermidis RP62a is expressed as number of cfu/biopsy

### Cytokine assays of the homogenates

The total homogenates, reduced by 120 µL for the quantitative culture, were diluted in 1 volume of lysis buffer (Greenberger M, J Immunol 155: 722-729, 1995) containing 1 % Triton X-100, 150 mM NaCl, 30 mM Tris, 2 mM CaCl₂, 2 mM MgCl₂, 0.2% aprotin and 0.2% leupeptin, pH 7.40, and incubated on ice for 1 h. Subsequently, the lysed homogenates were centrifuged at 130,000 x g for 15 min at 4 °C to remove cell debris. The cell-free supernatants were frozen at -80 °C, thawed, centrifuged at 5000 x g to remove macro-aggregates and stored in aliquots of 35 µL at -80 °C until use. Levels of interleukin (IL)-1β (R&D systems, Minneapolis, MN), IL-1α (R&D systems) and IL-4 (R&D systems) were measured by commercially available ELISA-kits, and expressed as picograms (pg) per mL homogenate.

### Statistical analysis

All values are expressed as Mean ± Std.Error. Two sample comparisons were made by analysis of variance (ANOVA) and comparisons between over time cytokine levels of groups by ANOVA in a general linear model - general factorial. The significance of differences between the frequencies of categorical variables was determined using the χ-square test. P<0.05 was considered significant.

### RESULTS

### Development of biomaterial-associated infection

In saline control mice no abscess formation was seen. In wild-type mice abscess formation, after injection of 10⁶ cfu of S. epidermidis, was observed around SEpvp at all time points tested, but not around SE segments. In contrast, IL-1R-/- mice abscesses were found neither around SE nor around SEpvp segments at any time point after inoculation with S. epidermidis (figure 9A).

In wild-type mice, at 2 days significantly more colonies were cultured from tissue surrounding SEpvp catheter segments (1,400 ± 1,200 cfu/biopsy) than from the tissue surrounding SE segments (180 ± 140 cfu/biopsy; P<0.01; figure 9B). Although the numbers of cfu decreased at 5 days and 14 days in all tissues (12/12) surrounding the SEpvp segments, these tissues remained culture-positive. Tissues surrounding SE were culture-negative at 5 and 14 days (figure 9B). Like in wild-type mice, in IL-1R-/- mice significantly more colonies were cultured from tissues surrounding SEpvp (2,200 ± 1,200 cfu/biopsy) than from tissues surrounding SE (800 ± 900 cfu/biopsy; P<0.05) at 2 days, and at 5 days all tissue homogenates surrounding SEpvp were positive while all tissue homogenates surrounding SE were negative. At 14 days however, 5 out of 6 tissue homogenates from SEpvp implantation sites were culture-negative in IL-1R-/-mice. Thus, significantly less (P<0.05) tissues were culture-positive in IL-1R-/- than in wild-type mice around SEpvp at 14 days. The cultured S. epidermidis had the same antibiogram as strain RP62a. No colonies were cultured from the tissue samples of mice injected with saline.

SEpvp segments implanted in wild-type mice were significantly more often culture-positive for S. epidermidis RP62a than SE segments (7 of 18 vs. 1 of 18; P=0.01; figure 9C). In IL-1R-/- mice similar results were obtained (8 of 18 vs. 3 of 18; P=0.02). The cultured S. epidermidis had the same antibiogram as strain RP62a. In wild-type mice tissues surrounding SE and SEpvp (7/18 and 18/18, respectively) were significantly more often culture-positive than the corresponding segments (1/18 and 7/18, respectively; P<0.05). Similarly, in IL-1-/- mice tissues surrounding SE and SEpvp (10/18 and 13/18, respectively) were significantly more often culture positive than the corresponding segments (3/18 and 8/18, respectively; P<0.05). This indicates persistence of S. epidermidis in tissue rather than on the catheter surface.

Thus, using an inoculum of 10⁶ cfu of S. epidermidis, in IL-1R-/- mice neither abscess formation nor persistent infection associated with SEpvp was induced, while wild-type mice were highly susceptible to SEpvp-associated abscess formation and infection.

Bacteria persisted in the tissue rather than that they adhered on the catheter segment.

### Histological examination of the inflammatory and foreign body reaction

Five histological features characteristic for the tissue inflammatory and foreign body response following the implantation of a foreign body were scored independently by 3 investigators who were blinded for the origin of the samples. The mean scores for these histological features at each time point are shown in figure 10. Variation of scores between investigators was always maximally 0.5 scale units.

The foreign body response around SE implanted in IL-1R-/- mice and wild-type mice showed only small differences (figures 10,11). In contrast, around SEpvp, the foreign body response was dramatically different in wild-type mice and in IL-1R-/- mice (figures 10,11). In wild-type mice, a prolonged and intensified inflammatory status was observed around SEpvp, characterized by the presence of purulence and a very strong infiltration up to 14 days, by strong fibrosis, and by delayed giant cell formation and delayed encapsulation of the foreign body. In contrast, around SEpvp implanted in IL-1R-/- mice, no purulence was present, a mild inflammation, weak fibrosis and a normal development of the foreign body reaction was observed, characterized by a layer containing giant cells bordering the implant at 5 days and presence of a fibrous capsule at 14 days (figures 10,11).

Hence, around SEpvp implanted in IL-1R-/- mice challenged with S. epidermidis a "normal" foreign body reaction, similar to that around SE implanted in wild-type mice challenged with S. epidermidis, was observed.

### Induction of IL-1β, IL-1α, and IL-4

IL-1β was detectable at low levels in saline control wild-type and IL1R-/- mice (figure 12). In wild-type mice, injected with S. epidermidis significantly higher IL-1β levels were found in tissue surrounding SEpvp segments than in tissue surrounding SE (p<0.01 for the over time IL-1β profile). In IL-1R-/- mice at 2 days, significantly higher IL-1β levels were found in tissue surrounding the implanted SE (1,624 ± 529 vs 545 ± 211; p=0.002) and in tissue surrounding SEpvp (5,069 ± 2,478 vs. 1,418 ± 763; p =0.007), than in wild-type mice. At later time points no differences in IL-1β levels between wild-type and IL-1R-/- were detected around SEpvp segments. In tissues surrounding SE in wild-type and IL-1R-/-, IL-1β levels were equal at 5 days, but at 14 days levels in IL-1R-/- mice were higher again (p<0.01).

IL-1α was detectable in saline control wild-type and IL-1R-/- mice (figure 12). In mice inoculated with S. epidermidis, IL-1α levels around the implanted SE and SEpvp segments increased up to 5 days and remained elevated up to 14 days. No differences were found between IL-1α levels in tissues surrounding SE and SEpvp, nor in tissues from wild-type and IL-1R-/-mice.

IL-4 was detectable in saline control wild-type and IL-1R-/- mice (figure 12). In wild-type mice injected with S. epidermidis, IL-4 levels were increased in tissue around SE after 5 days, whereas in tissue surrounding SEpvp no increase was found. The over time IL-4 profile in tissue surrounding SE was significantly higher (p=0.025; figure 12) than in tissue surrounding SEpvp. In tissues surrounding either SE or SEpvp segments from IL-1R-/- mice significantly higher levels of IL-4 were detected than in wild-type mice (both p<0.01; figure 12).

### CONCLUSION

In the present Example, the role of locally produced IL-1 in the pathogenesis of BAI was investigated. It was shown that not only IL-1β, but also IL-1α concentrations are elevated in tissues around infected biomaterials. Moreover, it was demonstrated that IL-1R-/- mice had no abscess formation and were less susceptible to persistent S. epidermidis infection associated with SEpvp catheters than wild-type mice. The foreign body reaction around SEpvp was delayed in wild-type mice but not in IL-1R-/- mice. These data demonstrate that IL-1 plays a detrimental role in this experimental model for BAI.

In conclusion, BAI in the SEpvp model is associated with an exaggerated and protracted local IL-la and IL-1β response. Blocking IL-1 effects results in a reduced susceptibility to BAI and a normal foreign body reaction, suggesting a role for high IL-1 levels in compromising the host by stimulating bacterial persistence, and in inhibiting IL-4 production. The results of this study demonstrate an important role for the host response to the various biomaterials in the pathogenesis of BAI, rather than for the extent of adherence of bacteria to biomaterial. Local inhibition of IL-1 activity according to the invention, for example by means of an IL-1 antagonist, thus is of benefit to the host as an adjunctive therapy for infections associated with biomaterials.

## Claims

1. Use of one or more immunomodulators for the preparation of a medicament for the prevention and/or treatment of biomaterial-associated infections in humans or animals.

2. Use as claimed in claim 1, wherein the immunomodulator is a compound that stimulates the host defense of the human or animal.

3. Use as claimed in claim 1 or 2, wherein the immunomodulator is a compound that stimulates intracellular killing of the infecting agent.

4. Use as claimed in claims 1-3 wherein the immunomodulator is a cytokine, its receptor antagonist, a cytokine derivative, or cytokine analogue.

5. Use as claimed in claim 4, wherein the immunomodulator is selected from the group consisting of interferon-γ (IFN-γ), interleukin-12 (IL-12), IL-15, interleukin-18 (IL-18), and interleukin-1 receptor antagonist (IL-1 RA).

6. Use as claimed in claims 1-3 wherein the immunomodulator is a CpG oligonucleotide.

7. Use as claimed in claims 1-6, wherein the infection are bacterial infections.

8. Use as claimed in claim 7, wherein the bacteria are coagulase-negative staphylococci, such as Staphylococcus epidermidis, Staphylococcus aureus, Entercocci, Corynebacterium spp., Serratia spp., Klebsiella spp., Bacillus spp., Proteus spp., Enterobacter spp., Enterobacteriaceae spp., Pseudomanas spp., Acinetobacter spp., Clostridium spp. or Diftroide spp...

9. Use as claimed in claim 1-6, wherein the infections are yeast infections.

10. Use as claimed in claim 9, wherein the yeasts are Candida spp. or Malezzia spp.

11. Use of interferon-γ for the preparation of a medicament for the prevention and/or treatment of biomaterial-associated infections in humans or animals.

12. Use as claimed in claim 10, wherein the infections are caused by Staphylococcus epidermidis.

13. Use as claimed in claims 1-12, wherein the medicament is a systemically acting medicament.

14. Use as claimed in claim 13, wherein the medicament is intended for oral, intravenous, subcutaneous, intramusculair administration.

15. Use as claimed in claims 1-12, wherein the medicament is a topically acting medicament.

16. Use as claimed in claim 15, wherein the medicament takes the form of a injection fluid, plaster, > ointment, cream, gel, coating on the biomaterial, or a hydrogel.

17. Biomaterial provided with one or more immunomodulators for the prevention and/or treatment of infections in humans or animals associated with the biomaterial.

18. Biomaterial as claimed in claim 17, wherein the biomaterial is coated with the immunomodulator.

19. Biomaterial as claimed in claim 18, wherein the immunomodulator is comprised in a hydrogel coating of the biomaterial.

20. Biomaterial as claimed in claim 17, wherein the biomaterial is impregnated with the immunomodulator.

21. Biomaterial as claimed in claims 17-20, wherein the immunomodulator is a compound that stimulates the host defence of the human or animal.

22. Biomaterial as claimed in claim 17-21, wherein the immunomodulator is a compound that stimulates intracellular killing of the infecting agent.

23. Biomaterial as claimed in claim 17-22 wherein the immunomodulator is a cytokine, its receptor antagonist, a cytokine derivative, or cytokine analogue.

24. Biomaterial as claimed in claim 23, wherein the immunomodulator is selected from the group consisting of interferon-γ (IFN-γ), interleukin-12 (IL-12), IL-15 interleukin-18 (IL-18), and interleukin-1 receptor antagonist (IL-1 RA).

25. Biomaterial as claimed in claims 17-22 wherein the immunomodulator is a CpG oligonucleotide.

26. Biomaterial as claimed in claims 17-25 wherein the biomaterial is selected from the group consisting of silicon elastomer, polyamide and their surface-modified polyvinylpyrrolidone-grafted derivatives.

27. Biomedical device made of a biomaterial as claimed in claims 17-26.
